# EUROPEAN PATENT SPECIFICATION

(11) **EP 1 026 256 B1**
(45) Date of publication and mention of the grant of the patent: **11.08.2004**
(21) Application number: 00300853.9
(22) Date of filing: 03.02.2000
(51) Int. Cl.: C12P 13/00, C12P 41/00, C12N 9/88

(54) **Method for the production of optically active cyanohydrins**
Verfahren zur Herstellung von optisch aktiven Cyanhydrinen
Procédé de production de cyanhydrines optiquement actives

(30) Priority: 03.02.1999 JP 2564099
(43) Date of publication of application: 09.08.2000
(73) Proprietor: Nippon Shokubai Co., Ltd., Osaka-shi, Osaka 541-0043 (JP)
(72) Inventor: Semba, Hisashi, Tsuchiura-shi, Ibaraki 300-0810 (JP)
(74) Representative: Maschio, Antonio, Dr.

(56) References cited:
- PÖCHLAUER, P.: "Syntheses of Homochiral Cyanohydrins in An Industrial Environment: Hxdroxy Nitrile Lyases Offer New Options." CHIMICA OGGI, vol. 16, no. 5, 1998, pages 15-19, XP000915454
- HASSLACHER, M. ET AL.: "High-Level Intracellular Expression of Hydroxynitrile Lyase from the Tropical Rubber Tree Hevea brasiliensis in Microbial Hosts." PROTEIN EXPRESSION PURIF., vol. 11, no. 1, 1997, pages 61-71, XP000915456
- TRUMMLER, K. ET AL.: "Expression of the Zn2+ -containing hydroxynitrile lyase from flax (Linum usitatissimum) in Pichia pastoris - utilization of the recombinant enzyme for enzymatic analysis and site-directed mutagenesis." PLANT SCIENCE, vol. 139, 1998, pages 19-27, XP000915455
- JOHNSON, D.V. ET AL.: "The Chemoenzymatic Synthesis of (S)-13-Hydroxyoctadeca-(9Z, 11E)-dienoic Acid using the Hydroxynitrile Lyase from Hevea brasiliensis." TETRAHEDRON, vol. 53, no. 2, 1997, pages 617-624, XP004105164

## Description

### FIELD OF THE INVENTION

The present invention relates to a method for the production of optically active cyanohydrins.

### BACKGROUND OF THE INVENTION

Optically active cyanohydrins are useful as optically active intermediates for producing insecticides such as pyrethroids or as an intermediates for synthesizing drugs. Various methods for synthesizing optically active cyanohydrins have been proposed heretofore. However, these methods have practical problems and no method has yet been established for directly synthesizing the optically active cyanohydrins for industrial purposes.

For example, a method for synthesizing optically active cyanohydrins as intermediates for producing pyrethroid insecticides is known. According to this method, racemic cyanohydrin ester is chemically synthesized as a starting material, and subjected to asymmetric hydrolysis using an enzyme (JP-A-61-5794, JP-A-62-65688). This method, however, is associated with problems such as multiple production steps, and the necessity of degradation, racemization and recycling of enantiomer ester that remains after the asymmetric hydrolysis reaction.

An enzyme was recently reported which synthesized optically active cyanohydrin alone directly from aldehyde or ketone and hydrocyanic acid, and methods have been studied for synthesizing optically active cyanohydrins using this enzyme. For example, methods for synthesizing R-cyanohydrins by using R-hydroxynitrile lyase (EC4.1.2.10) derived from almond (*Prunus amigdalus*) or R-hydroxynitrile lyase derived from flax (*Linum usitatissimum*), and methods for synthesizing S-cyanohydrins by using S-hydroxynitrile lyase (EC4.1.2.11) derived from sorghum (*Sorghum bicolor*), S-hydroxynitrile lyase (EC4.1.2.37) derived from cassava (*Manihot esculenta*), or S-hydroxynitrile lyase (EC4.1.2.39) derived from tropical rubber tree (*Hevea brasiliensis*) are known. All of the enzymes used in the above methods are obtained by being extracted from plant tissues or by immobilizing such enzymes extracted from the plant tissues. Enzyme extraction from plant tissues, however, is not suitable for industrial purposes since extraction and purification are complicated, troublesome and associated with a great amount of enzyme loss. Moreover, for immobilizing the extracted enzyme, the cost of the carriers for immobilization cannot be neglected and thus such immobilized enzyme is also not suitable for industrial use, while use of inexpensive reaction catalyst is requisite.

Methods for synthesizing optically active cyanohydrins by using recombinant cells transformed with enzyme genes (hereinafter, often referred to as "recombinant cells") are also known. These methods use, for example, recombinant cells transformed with S-hydroxynitrile lyase (EC4.1.2.39) gene from tropical rubber tree (*Hevea brasiliensis*) (WO 98/30711) or recombinant cells transformed with S-hydroxynitrile lyase (EC4.1.2.37) gene from cassava (*Manihot esculenta*) (Angrew, Chem. Int. Ed. Engl. 35, 437-439, 1996; Biotechnol. Bioeng. 53, 332-338, 1997; JP-A-9-227488). According to these methods, the enzymes need to be extracted from the recombinant cells which is as troublesome as the above-described process of extracting the enzymes from the plant tissue.

Pöchlauer, P. describes isolating S-hydroxynitrole lyase from a variety of fermenting recombinant microorganisms which have been transformed with its encoding gene obtained from the rubber plant *Hevea brasiliensis* (Pöchlauer, P., 1998, *Chemica* OGGI, 16: 15-19). The isolated recombinant enzyme was then incorporated into a conventional stirred tank reactor capable of operating in an aqueous and an organic environment for optimised production of cyanohydrins.

Thus, the present invention aims at providing a method for efficiently producing optically active cyanohydrins at low cost.

### SUMMARY OF INVENTION

The present inventor has undergone intensive studies to solve the above-described problems, and found, for the first time, that a recombinant cell transformed with a hydroxynitrille lyase gene can itself be used in a like manner to the enzyme isolated therefrom for synthesizing optically active cyanohydrins, and that the use of this cell improves the stability of the enzymatic activity as compared to the use of the enzyme isolated therefrom, thereby accomplishing the present invention.

Thus, the present invention is a method for the production of optically active cyanohydrins, comprising adding recombinant microorganism cells which have been transformed by introducing an hydroxynitrile lyase enzyme gene and which retain an activity of the enzyme, to a reaction system comprising a reaction solvent containing one or more aldehydes or ketones and hydrogen cyanide or a substance capable of producing cyanide ions in the reaction system.

This specification includes all or part of the contents as disclosed in the specification of Japanese Patent Application No. 11-25640, which is a priority document of the present application.

### DETAILED DESCRIPTION OF THE INVENTION

Hereinafter, the present invention will be described in more detail.

According to the present invention, aldehydes or ketones as a reactive substrate may be represented by the following formula (I).

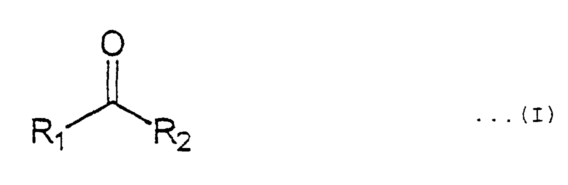

Wherein, R₁ and R₂ are (i) a hydrogen atom, (ii) a substituted or unsubstituted, linear or branched, saturated C₁₋₁₈ alkyl group, or (iii) a substituted or unsubstituted aromatic group having 5-22 cyclic members; where R₁ and R₂ do not represent hydrogen atoms at the same time.

With respect to (ii) above, when R₁ and R₂ represent substituted alkyl groups, the substituent is one or more of amino group, imino group, hydroxy group, C₁₋₈ alkoxy group, halogen, calboxyl group, C₃₋₂₀ cycloalkyl group, or aromatic group having carbon number of up to 22 which may be substituted with a heteroatom, N, O or S (here, when the substituent is a cyclic substituent, the substituent itself may be substituted with one or more halogen, hydroxy group, linear or branched C₁₋₈ alkyl group, or linear or branched C₂₋₈ alkenyl group).

With respect to (iii) above, the aromatic group may be a heteroaromatic group wherein up to 4 cyclic members are substituted with N, O and/or S. Where R₁ and R₂ represent substituted aromatic groups, the substituent may be one or more of amino group, imino group, hydroxy group, C₁₋₈ alkoxy group, allyloxy group, halogen, calboxyl group, linear or branched, saturated or unsaturated C₁₋₂₂ alkyl group (here, an aromatic group may be substituted with at least 2 substituents).

To convert the above-described aldehydes or ketones to optically active cyanohydrins, hydrogen cyanide is used as the substrate. The hydrogen cyanide may be supplied either in the form of liquid or gas. An aqueous solution of hydrogen cyanide, i.e., hydrocyanic acid (or prussic acid), may also be equally employed. Any substance that is capable of producing cyanide ion (CN⁻) upon addition to a reaction system may be used. Examples of such substances include salt of hydrogen cyanide such as sodium cyanide and potassium cyanide, or cyanhydrin compounds such as acetone cyanohydrin.

Recombinant microorganism cells which have been cultivated in suitable media and in which enzyme production has been induced are used as the catalyst of the reactions.

Specifically, examples of genes coding for hydroxynitrile lyase include genes coding for R-hydroxynitrile lyase derived from plants belonging to Rosaceae such as almond (*Prunus amigdalus*) (EC4.1.2.10) and blackberry (*Prunus serotina*); a gene coding for R-hydroxynitrile lyase derived from flax (*Linum usitatissimum*) belonging to Linaceae; a gene coding for S-hydroxynitrile lyase (EC4.2.2.11) derived from sorghum (*Sorghum bicolor*); a gene coding for S-hydroxynitrile lyase (EC4.1.2.37) derived from cassava (*Manihot esculenta*); a gene coding for S-hydroxynitrile lyase (EC4.1.2.39) derived from tropical rubber tree (*Hevea brasiliensis*); and a gene coding for S-hydroxynitrile lyase derived from ximenia *(Ximenia americana*).

The microorganisms that may be transformed by introducing the above-described enzyme genes are not specifically limited as long as they can incorporate and allow the above-mentioned enzyme genes to express and produce the enzymes. Such microorganisms are, for example, eukaryotes such as yeast and prokaryotes such as *E.coli*.

The recombinant microorganism may be used as a catalyst of the reaction in the form of wet cells directly obtained from the medium, or more preferably in the form of dry cells which are subjected to drying or dehydration before use to remove the excessive amount of moisture to enhance dispersibility in the reaction system.

The drying or dehydrating process of the cells is not specifically limited as long as the enzymatic activity of the cells is retained. Examples of such process include hot air drying, vacuum drying, lyophilization, spray drying, or drying with an organic solvent such as acetone.

The dry cells are powdered, or granulated by mixing with a binder. Alternatively, the dry cells may be mixed with insoluble carriers, or may be immobilized according to a known method.

Where the cells are used in a reaction solvent as a suspension, the reaction may be carried out by employing a batch or semi-batch system. On the other hand, a continuous system is advantageous where the cells used are filled in a filling tank that allows a liquid to flow therethrough.

When a large amount of water is present in the reaction system, the optically active cyanohydrins resulting from the enzyme reactions are likely to be racemizated, or use of aldehydes or ketones having low solubility to water as the raw material will decrease the production efficiency. Accordingly, it is preferable that the reaction solvent mainly consists of an organic solvent that has poor water solubility or are water-immiscible. Such organic solvent is not specifically limited as long as it does not influence the enzymatic reaction for synthesizing optically active cyanohydrins, and may suitably be selected depending upon physical properties of the substrates (i.e., aldehydes or ketones) used for the synthesis reaction and upon the physical properties of the cyanohydrins to be produced. Specifically, examples of such organic solvent include linear or branched or cyclic, saturated or unsaturated, aliphatic or aromatic hydrocarbon solvent which may optionally be halogenated, such as pentane, hexane, toluene, xylene, methylene chloride and the like; linear or branched or cyclic, saturated or unsaturated, aliphatic or aromatic alcohol solvent which may optionally be halogenated, such as isopropyl alcohol, n-butanol, isobutanol, t-butanol, hexanol, cyclohexanol, n-amyl alcohol and the like; linear or branched or cyclic, saturated or unsaturated, aliphatic or aromatic ether solvent which may optionally be halogenated, such as diethylether, dipropylether, diisopylether, dibutylether, methyl-t-buthylether and the like; and linear or branched or cyclic, saturated or unsaturated, aliphatic or aromatic ester solvent which may optionally be halogenated, such as methyl formate, methyl acetate, ethyl acetate, butyl acetate, methyl propionate and the like. These solvents may be used alone, or two or more of them may be used in combination. These organic solvents may be saturated with an aqueous buffer of pH 7 or less such as a citrate buffer, a phosphate buffer or an acetate buffer.

The concentration of the aldehyde or ketone contained in the reaction solvent is preferably within the range of 0.01 mM to 5 M. Hydrogen cyanide or the substance capable of producing cyanide ion in the reaction system is used for 1-20 mol per mol of aldehyde or ketone, and the recombinant microorganism cell is used for an amount that allows an enzymatic activity of 1 unit/mmol or more to the concentration of aldehyde or ketone.

The enzymatic activity of the cells may be calculated as follows. The cells suspended in water or buffer are disrupted, and then subjected to centrifugation to obtain the supernatant. Using the supernatant and DL-mandelonitrile as a substrate, the change of absorption at a wavelength of 249.6 nm upon benzaldehyde production resulting from degradation of the substrate with the enzyme is measured.

pH of the reaction solvent does not need to be adjusted if the above-described organic solvent is not saturated with an aqueous buffer. If the organic solvent is saturated with an aqueous buffer, the pH of the aqueous buffer is adjusted to be within the range of 3-7, preferably within the range of 3-6.

The reaction temperature is set as low as possible within a range that allows enzyme activity, generally 0-40°C, preferably 0-30°C, to suppress by-production of racemic cyanohydrin that is irrelevant to the enzymatic reaction.

Once the reaction is completed, the reaction solution and the cells are separated from each other to obtain a solution containing the reaction product. Components other than the optically active cyanohydrin are removed from the solution, thereby obtaining the optically active cyanohydrin of interest. The product is separated according to a routine method such as distillation, column chromatography, extraction or the like. Upon this separation, a dehydrating agent for dehydration, a stabilizer or the like may be added.

### EXAMPLES

Hereinafter, the present invention will be described in more details by way of examples.

### Example 1: Preparation of recombinant cells

### (1) Preparation of enzyme gene

Total mRNA was extracted from a leaf of cassava. The obtained mRNA was used as a template for cDNA synthesis to construct a cDNA library of cassava. The following PCR primers were synthesized with reference to the sequence information of cassava-derived S-hydroxynitrile lyase gene available from a publication (*Arch. Biochem. Biophys*. 311, 496-502, 1994).

Sense primer: GGG GAA TTC ATG GTA ACT GCA CAT TTT GTT CTG
ATT C (SEQ ID NO:1)

Antisense primer: GGG GTC GAC CTC ACG GAT TAG AAG CCG CCG
(SEQ ID NO:2)

Using these primers and the above-obtained cDNA as a template, PCR was conducted for a total of 35 cycles of: 95°C for 0.5 min.; 55°C for 0.5 min.; 72°C for 1 min. A gene corresponding to the gene coding for S-hydroxynitrile lyase was obtained. The obtained gene was analyzed for its sequence and was found to be consistent with the sequence described in the publication.

### (2) Preparation of enzyme gene recombinant cell

YEp352-GAP vector was obtained by introducing a GAP promoter and a terminator into YEp352 plasmid vector which is an *E.coli*/yeast shuttle vector containing an ampicillin-resistant gene, a sequence from yeast 2µ plasmid and a sequence of URA3 (a selective marker gene for the recombinant yeast). The cDNA of S-hydroxynitrile lyase obtained in (1) above was inserted between the GAP promoter and the terminator of the YEp352-GAP vector, thereby preparing yeast episomal expression vector YEp352-GC.

Using the expression vector YEp352-GC, host yeast cell, *Saccharomyces cerevisiae* Inv-Sc1 strain was transformed. Recombinant clones were selected in a uracil-free minimum selective medium having the following composition.

**Table 1**

| | |
|---|---|
| Yeast nitrogen base without amino acids (Difco) | 6.7 g/L |
| Glucose | 20 g/L |
| Adenine | 40 mg/ml |
| L-arginine | 20 mg/ml |
| L-aspartic acid | 100 mg/ml |
| L-glutamic acid | 100 mg/ml |
| L-histidine | 20 mg/ml |
| L-isoleucine | 30 mg/ml |
| L-lysine | 30 mg/ml |
| L-methionine | 20 mg/ml |
| L-phenylalanine | 50 mg/ml |
| L-serine | 375 mg/ml |
| L-threonine | 200 mg/ml |
| L-tryptophan | 40 mg/ml |
| L-tyrosine | 30 mg/ml |
| L-valine | 150 mg/ml |
| L-leucine | 60 mg/ml |

The recombinant yeast strain (YEp352-GC-S2 strain) obtained as described above was cultured for 24 hours in a YNBDCas liquid medium having the following composition.

**Table 2**

| | |
|---|---|
| Yeast nitrogen base without amino acids (Difco) | 6.7 g/L |
| Glucose | 20 g/L |
| Casamino acid | 20 g/L |
| L-tryptophan | 40 mg/ml |

The culture was centrifuged to collect the cells. The obtained wet cells were mixed with acetone, agitated and filtrated to obtain acetone-dried cells.

The enzymatic activities of the wet and dry cells were determined as follows.

The cells were added with 0.1 ml of glass beads (0.5 mm diameter) and frozen with liquid nitrogen. Then, 0.2 ml of 0.15 M sodium citrate buffer (pH 5) was added to the mixture, and the cells were disrupted by agitation. The solution containing the disrupted cells was subjected to centrifugation to obtain the supernatant for the determination of the enzymatic activities. The activities were determined, using DL-mandelonitrile as a substrate, as the change of absorption at a wavelength of 249.6 nm upon benzaldehyde production resulting from degradation of the substrate with the enzyme. The activity of degrading 1 µmol of the substrate per minute was assumed 1 unit.

According to the above-described determination, the enzymatic activity of the wet cells of the recombinant strain (YEp352-GC-S2 strain) of the invention was 200 unit/g (wet weight), while the enzymatic activity of the dry cells was 1,000 unit/g.

### Example 2

To 2.5 ml of diisopropylether, 120 mg of the acetone-dried cells prepared in Example 1, 0.5 mmol of benzaldehyde and 56.6 µl of hydrocyanic acid were added to allow synthesis reaction at room temperature (20-25°C) under agitation. After 24 hours, the reaction solution was analyzed by liquid chromatography under the following conditions:
Column: Chiralcel-OJ (Daicel Chemical Industries, Ltd.)
Column temperature: 25°C
Eluent: n-hexane:isopropyl alcohol = 80:20
Flow rate: 0.7 ml/min
Detection:
   (i) UV detector, wavelength 254 nm
   (ii) polarimeter
As a result, S-mandelonitrile with an optical purity of 94.1%ee was produced at a conversion rate of 89.7%.

### Example 3

The dry cells used in Example 2 were separated from the reaction solution and washed with diisopropylether. To 2.5 ml of diisopropylether. 120 mg of the washed cells, 0.5 mmol of benzaldehyde and 56.6 µl of hydrocyanic acid were added, and the resultant mixture was subjected to synthesis reaction at room temperature (20-25°C) under agitation. After 24 hours, the reaction solution was analyzed by liquid chromatography as described above. It was found that S-mandelonitrile with an optical purity of 93.6%ee was produced at a conversion rate of 71%. The reaction was repeated under the same conditions by recycling only the cells. The enzymatic activity of the cells did not decrease after a total of 5 times of reactions.

### Comparative Example 1

To 2.5 ml of diisopropylether, an immobilized enzyme [prepared by mixing 75 µl of enzymatic solution (491.3 unit/ml) of partially purified enzyme produced from enzyme gene recombinant *E.coli*, with 75 mg of avicel cellulose], 0.5 mmol of 3-phenoxybenzaldehyde and 55.6 mg of hydrocyanic acid were added, and the mixture was subjected to synthesis reaction at room temperature (20-25°C) under agitation. After 24 hours, the reaction solution was analyzed by liquid chromatography as described above, and it was found that S-3-phenoxybenzaldehyde cyanohydrin with an optical purity of 92%ee was produced at a conversion rate of 62%. The reaction was repeated under the same conditions by recycling the immobilized enzyme. As a result, the conversion rate and the optical purity decreased to 24% and 22%ee, respectively upon the second analysis, and no enzymatic activity was detected upon the third analysis.

### Example 4

The wet cells of the recombinant yeast strain (YEp352-GC-S2 strain) collected in Example 1 were subjected to lyophilization to obtain lyophilized cells. To 2.5 ml of diisopropylether, 120 mg of the lyophilized cells, 0.5 mmol of benzaldehyde and 56.6 µl of hydrocyanic acid were added, and the resultant mixture was subjected to synthesis reaction at room temperature (20-25°C) under agitation. After 45 hours, the reaction solution was analyzed by liquid chromatography as described above. As a result, it was found that S-mandelonitrile with an optical purity of 65.1%ee was produced at a conversion rate of 65%.

### Example 5

To 2.5 ml of diisopropylether which had been saturated with 0.15 M sodium citrate buffer (pH 4), 120 mg of the acetone-dried cells prepared in Example 1, 1 mmol of 3-phenoxybenzaldehyde and 300 µl of hydrocyanic acid were added, and the resultant mixture was subjected to synthesis reaction at room temperature (20-25°C) under agitation. After 60 hours, the reaction solution was analyzed by liquid chromatography as described above. As a result, it was found that S-3-phenolxybenzaldehyde cyanohydrin with an optical purity of 58.6%ee was produced at a convertion rate of 96.9%.

According to the present invention, optically active cyanohydrin can easily and efficiently be synthesized by using a hydroxynitrile lyase gene recombinant cell itself without the need of extracting hydroxynitrile lyase therefrom.

## Claims

1. A method for producing optically active cyanohydrins, comprising adding recombinant microorganism cells which have been transformed by introducing a hydroxynitrile lyase enzyme gene and which retain an activity of the enzyme, to a reaction system comprising a solvent containing aldehydes or ketones, and hydrogen cyanide or a substance capable of producing cyanide ions in the reaction system.

2. A method for producing optically active cyanohydrins according to claim 1, wherein the recombinant microorganism cells are obtained by drying or dehydrating cultured cells.

3. A method for producing optically active cyanohydrins according to claim 1, wherein the reaction solvent is an organic solvent which is poorly-soluble in or immiscible with water.

## Patentansprüche

1. Verfahren zur Herstellung optisch aktiver Cyanohydrine, bei dem man rekombinante Mikroorganismenzellen, die durch Einführung eines Gens für das Enzym Hydroxynitrillyase tranformiert wurden und die eine Aktivität des Enzyms behalten, einem Reaktionssystem zusetzt, das ein Lösungsmittel umfaßt, welches Aldehyde oder Ketone und Cyanwasserstoffsäure oder eine Substanz, die Cyanidionen in dem Reaktionssystem bilden kann, umfaßt.

2. Verfahren zur Herstellung optisch aktiver Cyanohydrine nach Anspruch 1, bei dem man die rekombinanten Mikroorganismenzellen durch Trocknen oder Dehydrieren kultivierter Zellen erhält.

3. Verfahren zur Herstellung optisch aktiver Cyanohydrine nach Anspruch 1, bei dem das Reaktionslösungsmittel ein organisches Lösungsmittel ist, welches in Wasser schlecht löslich oder mit Wasser unmischbar ist.

## Revendications

1. Procédé pour la production de cyanhydrines optiquement actives, comprenant l'addition de cellules de microorganismes recombinantes qui ont été transformées par introduction d'un gène d'enzyme hydroxynitrile-lyase et qui conservent une activité de l'enzyme, à un système réactionnel comprenant un solvant contenant des aldéhydes ou cétones et du cyanure d'hydrogène ou une substance capable de produire des ions cyanure dans le système réactionnel.

2. Procédé pour la production de cyanhydrines optiquement actives suivant la revendication 1, dans lequel les cellules de microorganismes recombinantes sont obtenues par séchage ou déshydratation de cellules en culture.

3. Procédé pour la production de cyanhydrines optiquement actives suivant la revendication 1, dans lequel le solvant de réaction est un solvant organique qui est faiblement soluble dans l'eau ou non miscible à l'eau.
